(19) Europäisches Patentamt
European Patent Office
Office européen des brevets

(11) Publication number: **0 227 355 B1**

(12) **EUROPEAN PATENT SPECIFICATION**

(45) Date of publication of patent specification: **25.03.92**

(51) Int. Cl.[5]: **C07D 498/18**, A61K 31/395, //(C07D498/18,311:00,273:00, 221:00)

(21) Application number: **86309449.6**

(22) Date of filing: **04.12.86**

Divisional application 91200118.7 filed on 04/12/86.

(54) **Prodrugs of rapamycin.**

(30) Priority: **06.12.85 US 806152**

(43) Date of publication of application:
**01.07.87 Bulletin 87/27**

(45) Publication of the grant of the patent:
**25.03.92 Bulletin 92/13**

(84) Designated Contracting States:
**AT BE CH DE ES FR GR IT LI LU NL SE**

(56) References cited:
**EP-A- 0 041 795**
**EP-A- 0 046 661**

(73) Proprietor: **THE UNIVERSITY OF KANSAS**
**226 Strong Hall**
**Lawrence, Kansas 66045-2300(US)**

(72) Inventor: **Stella, Valentino John**
**777 Sunset Drive**
**Lawrence Kansas(US)**
Inventor: **Kennedy, Paul Edwin**
**5101 River Road**
**Bethesda Maryland(US)**

(74) Representative: **Wileman, David Francis Dr. et al**
**c/o Wyeth Laboratories Huntercombe Lane South**
**Taplow Maidenhead Berkshire SL6 OPH(GB)**

**EP 0 227 355 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to water soluble prodrugs of rapamycin in the form of pharmaceutically acceptable salts and in particular to certain acylamino derivatives of rapamycin such as, for example, the glycinate prodrugs of rapamycin.

Rapamycin is a known compound described and claimed in United States Letters Patent Nos 3,929,992, issued December 30, 1975, and 3,993,749 issued November 23, 1976. Moreover, certain of its acyl derivatives are disclosed and claimed in United States Letters Patent No 4,316,885, issued February 23, 1982.

Rapamycin has been disclosed and claimed as useful in the treatment of tumors in Belgian Patent No. 877,700. Rapamycin is, however, only very slightly soluble in water, ie 20 micrograms per milliliter, and special injectable formulations have been developed for administration to patients, such as those described and claimed in European Patent Number EP 41,795. These formulations are not altogether satisfactory for a number of reasons including toxicity of the carrier. Accordingly, there is a need in the art for a rapamycin derivative or prodrug which is relatively soluble in water so as to to form a safe injectable solution and which is as effective as rapamycin in the treatment of tumors.

It has now been found that water soluble prodrugs of rapamycin can be synthesized which decompose into products including rapamycin in the presence of human plasma and animal tissue homogenates. Such prodrugs of rapamycin provide a component of a valuable pharmaceutical injectable composition for the treatment of tumor in humans.

The water soluble prodrugs of this invention comprise pharmaceutically acceptable salts of disubstituted derivatives at positions 28 and 43 of the rapamycin structure. The assignments are based on a structural elucidation published by Findlay et al in Can. J. of Chem 58, 579 (1980). This structure is shown below:

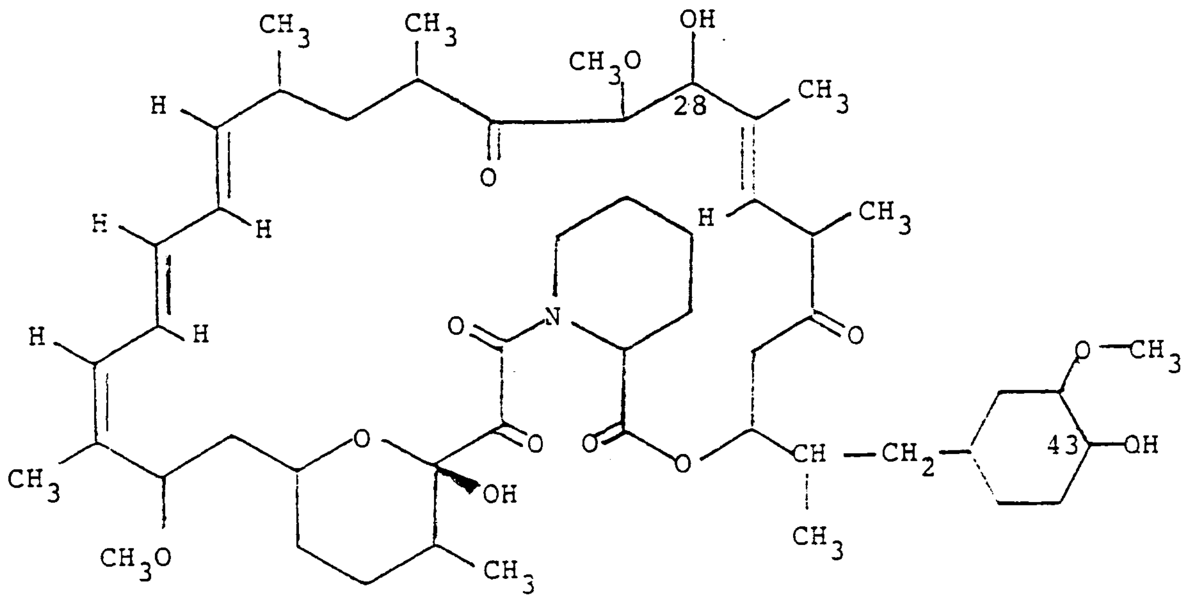

Rapamycin

The di-substituted derivatives are those having a substituent at positions 28 and 43 of the rapamycin structure having the following configuration

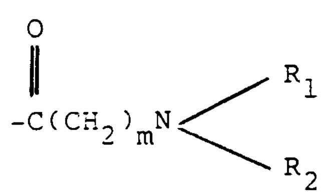

wherein m is an integer from 1 to 3, wherein $R_1$ and $R_2$ are each hydrogen or an alkyl radical having from one to three carbon atoms or wherein $R_1$ and $R_2$ together with the nitrogen atom to which they are attached

form a saturated heterocyclic ring having four to five carbon atoms.

Compounds in which $R_1$ and $R_2$ are each an alkyl radical of one to three carbon atoms are preferred.

Examples of $R_1$ and $R_2$ are methyl and ethyl. Examples of m are 1 and 2.

This invention also provides processes for preparing pharmaceutically acceptable salts of the di-substituted water soluble prodrugs of rapamycin.

Accordingly this invention provides a process for preparing a pharmaceutically acceptable salt of a water-soluble rapamycin derivative disubstituted at positions 28 and 43 by an acylamino substituent having the configuration

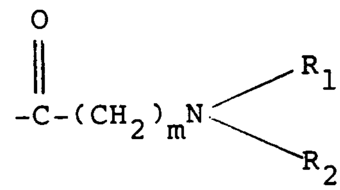

wherein m, $R_1$ and $R_2$ are as defined above which comprises acylating rapamycin with a acylating agent containing the said acylamino substituent having the configuration

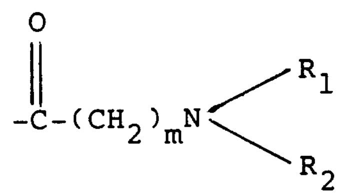

and if required acidifying the product to give a pharmaceutically acceptable salt.

The acylation may be carried out by standard acylation procedures which preferably foster neutral conditions. The acylating agent may be the acid, the acid halide (ie the chloride or bromide), the acid anhydride or an activated ester of said acylamino substituent. The acid form of the acylamino substituent is preferred as an acylating agent in the presence of a suitable coupling agent. The particular coupling agent may be most effective in the presence of a catalyst and/or an acid scavenger. Examples of preferred coupling agents are N,N'-dicyclohexylcarbodiimide, 1,1'-carbonyldiimidazole, diethylazodicarboxylate, 2,2'-dithiopyridine and N,N-diisopropylcarbodiimide. Diethylazodicarboxylate and 2,2'-dithiopyridine require the use of a catalyst such as triphenylphosphine. With these two coupling agents and triphenylphosphine as a catalyst a non-chlorinated solvent, such as an anhydrous ether, for example tetrahydrofuran, is preferable. With the other coupling agents the use of an acid scavenger, such as 4-dimethylaminopyridine or 4-pyrrolidinopyridine, is commonly preferred. With these coupling agents and catalysts a solvent such as anhydrous methylene chloride or chloroform may be used.

With the acid halide (preferably the acid chloride) a tertiary amine such as pyridine or triethylamine is preferred as an acid scavenger type catalyst and a solvent such as anhydrous methylene chloride or chloroform may be used.

In a preferred embodiment the acylation is carried out by reacting rapamycin with an acid of formula

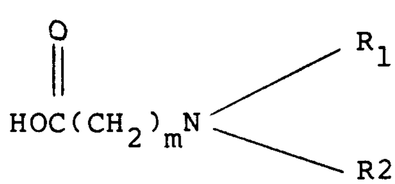

III

in the presence of a coupling agent, eg a carbodiimide (such as dicyclohexylcarbodiimide or diisopropylcarbodiimide) or carbonyldiimidazole.

A catalyst such as 4-dimethylaminopyridine or 4-pyrrolidinopyridine is preferred for use with such coupling agents. Coupling agents including carbodiimides and methods using them are well known in the art since they are extensively used in peptide chemistry - see for example E. Schroder and K. Lubke "The Peptides" Vol. 1, Acadamic Press, New York and London 1965 and Mr Bodanszky and M A Ondetti, Peptide Synthesis 1966 Interscience USA.

Where one or both of $R_1$ and $R_2$ is hydrogen, protection of the amine function of the acylating agent by a protecting group which is removable under neutral conditions is preferred. For example, where one or both $R_1$ and $R_2$ is hydrogen, the amine function of such acylating agent may be protected by using the acid chloride hydrochloride or the acylamino substituent as the acylating agent.

The acid chloride hydrochloride acylating agent may be made in a known manner by reacting the acid form of the acylating agent with one equivalent of hydrogen chloride gas then, with one equivalent phosphorous pentachloride. These reactions may be carried out in anhydrous methylene chloride. The product acid chloride hydrochloride may be recovered as a precipitate from toluene, hexane or cyclohexane. The acylation using said acid chloride hydrochloride may be carried out in a known manner using a weak base such as pyridine urea, dimethylaniline or trimethylamine as acid scavenger.

This invention also provides an injectable pharmaceutical composition comprising a water-soluble pharmaceutically acceptable salt of a derivative of rapamycin as defined above and a pharmaceutically acceptable carrier. Water or any water-based carrier known in the art can be used, eg distilled water, water containing 5% by weight dextrose or a physiologically acceptable salt solution. Such physiologically acceptable salt solution should have a pH in the neutral range, as for example, normal saline or Lactate Ringers solutions.

The preparation of typical water soluble prodrugs of rapamycin of this invention is illustrated in the Examples which were carried out using the following procedures. Example 1 illustrates the preparation of the free base.

## EXAMPLE 1

Synthesis of Bis-N,N-Dimethylglycinate Ester of Rapamycin

In a dry 100 mL round bottom flask was placed 2.80 g($3.07 \times 10^{-3}$ moles) of rapamycin, 0.616 g ($5.98 \times 10^{-3}$ moles) of N,N-dimethylglycine and 1.40 g ($6.80 \times 10^{-3}$ moles) of dicyclohexylcarbodiimide. The flask was placed under a nitrogen atmosphere and 60 mL of anhydrous methylene chloride (dried over $P_2 0_5$) was added followed by 60 mg of 4-dimethylaminopyridine. The reaction was stirred overnight at room temperature. A thin layer chromatogram (TLC) of the reaction (solvent system 1:1 acetone:methylene chloride) was taken and indicated the reaction to be complete. Some bisglycinate was present at a Rf of 0.09.

The reaction was worked-up by first filtering off the dicyclohexylurea (DCU). The solvent was removed on the rotovapor to give a white solid. The crude product was chromatographed on 18gm of silica gel using 300 mL of ethyl acetate to elute rapamycin plus residual DCU. The monoglycinate derivative was eluted with 1:1 methylene chloride:acetone to give 1.67 g of product, yield 55%. This material was found difficult to recrystallize. NMR (300 MHZ, solvent $CDCl_3$) indicated the spectrum of the prodrug to be practically identical to that of rapamycin except for the two singlets arising from the glycinate group. The N,N dimethyl protons appeared as a singlet at $\delta$ 2.32. The methylene group of the glycinate was found at $\delta$ 3.16 as a singlet.

## EXAMPLE 2

Synthesis of Bis N,N-Dimethylglycinate Ester of Rapamycin

The bis-glycinate prodrug of rapamycin substituted at positions 28 and 43 of the rapamycin structure was synthesized by the addition of 1 eq of rapamycin, 3 eq of N,N-dimethylglycine, 3.3 eq. of dicyclohexylcarbodiimide and 0.16 eq. of 4-N,N-dimethylaminopyridine. After purification on silica gel, 64% of bis-glycinate was obtained. NMR confirmed the product with two 6 proton singlets for the methyl groups of the two glycinate groups.

The formation of the methane sulfonic acid salt of the bis-glycinate was accomplished in 92% yield by the addition of 1.95 eq of methane sulfonic acid. The use of two equivalents caused the decomposition of the prodrug.

## EXAMPLE 3

### Synthesis of Bis-3-(N,N-Diethylamino)propionate ester of rapamycin, hydrochloride salt

In a dry 100 mL round bottom flask was placed 1.00g ($1.09 \times 10^{-3}$ moles) or rapamycin, 0.34 g ($2.16 X 10^{-3}$ moles) N,N-diethylaminopropionic acid hydrochloride salt and 0.50 g ($2.43 \times 10^{-3}$ moles) of dicyclohexylcarbodiimide.

The vessel was placed under a nitrogen atmosphere and 25 mL of anhydrous methylene chcloride (dried over $P_2O_5$ ) was added followed by 15 mg of 4-dimethylaminopyridine. The reaction was stirred overnight at room temperature. The next day a TLC of the reaction (solvent system: ethyl acetate) on silanized silica gel plate was taken and indicated the reaction to be complete.

The $R_f$ of the monopropionate hydrochloride salt of rapamycin was 0.34 and 0.01 for the bispropionate hydrochloride salt which was also formed in the reaction. The dicyclohexylurea was filtered from the reaction and the solvent removed on the rotovapor. The crude product was chromatographed on 12 g of silanized silica gel. The column was first developed with 200 mL of ethyl acetate to remove any rapamycin and also residual dicyclohexylurea. The product was eluted with ethyl acetate to give 0.61 g of the monopropionate derivative, yield 53%. This compound was found difficult to recrystallize and unstable to prolonged exposure to light. NMR (200 MHz, solvent $CDCL_3$) indicated the spectrum of the prodrug to be practically identical with that of rapamycin. The propionate group did not give sharp easily interpreted resonances as was the case with the glycinate prodrug. This is the result of the resonances being multiplets resulting from the ethyl groups which are not as easily seen among the other resonances from rapamycin. Broad peaks did appear around $\delta$ 1.2 and $\delta$ 1.5 which were not found in rapamycin.

## Claims

### Claims for the following Contracting States : BE, CH, DE, FR, IT, LI, LU, NL, SE

1. A water soluble pharmaceutically acceptable salt of a derivative of rapamycin which is disubstituted at positions 28 and 43 of rapamycin with the substituent having the configuration:

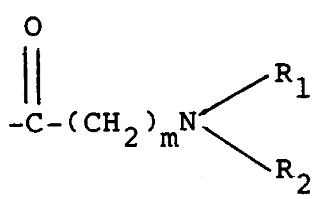

wherein m is an integer from 1 to 3,
wherein $R_1$ and $R_2$ are each hydrogen or an alkyl radical having from one to three carbon atoms or wherein $R_1$ and $R_2$ together with the nitrogen to which they are attached form a saturated heterocyclic ring having four to five carbon atoms.

2. A rapamycin derivative as claimed in Claim 1 wherein $R_1$ and $R_2$ are each an alkyl radical of 1 to 3 carbon atoms.

3. A rapamycin derivative as claimed in Claim 2 wherein $R_1$ and $R_2$ are methyl or ethyl.

4. A rapamycin derivative as claimed in any one of Claims 1 to 3 wherein m is 1 or 2.

5. The di-substituted derivative of Claim 1 wherein the substituent is

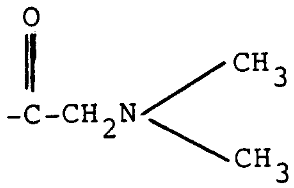

6. The methanesulphonate salt of the compound claimed in Claim 5

7. A process for preparing a water soluble pharmaceutically acceptable salt of a derivative of rapamycin which is disubstituted at positions 28 and 43 by an acylamino substituent having the configuration

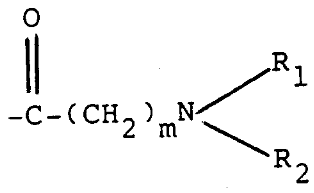

wherein m, $R_1$ and $R_2$ are as defined in Claim 1, which comprises acylating rapamycin with an acylating agent containing the acylamino substituent having the configuration

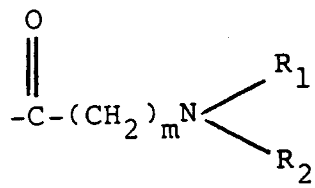

and if required acidifying to form a pharmaceutically acceptable salt.

8. A process as claimed in Claim 7 wherein the acylating agent is the acid, the acid halide, the acid anhydride or an activated ester of said acylamino substituent.

9. A process as claimed in Claim 8 wherein the acylating agent is the acid of said acylamino substituent in the presence of a coupling agent.

10. A process as claimed in Claim 9 wherein the coupling agent is N, N'-dicyclohexylcarbodiimide, 1,1'-carbonyldiimidazole, diethylazodicarboxylate, 2,2'-dithiopyridine, or N,N-diisopropylcarbodiimide.

11. A process as claimed in any one of Claims 7 to 10 wherein $R_1$ and $R_2$ are each alkyl of 1 to 3 carbon atoms.

12. An injectable pharmaceutical composition comprising a water soluble derivative of rapamycin as claimed in any one of Claims 1 to 6 and a pharmaceutically acceptable carrier.

**Claims for the following Contracting States : AT, GR, ES**

1. A process for preparing a water soluble pharmaceutically acceptable salt derivative of rapamycin which is disubstituted at positions 28 and 43 of rapamycin with a acylamino substituent having the configuration:

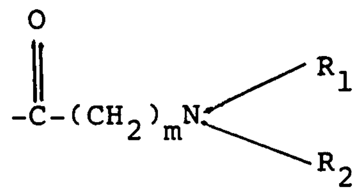

wherein m is an integer from 1 to 3,
wherein $R_1$ and $R_2$ are each hydrogen or an alkyl radical having from one to three carbon atoms or wherein $R_1$ and $R_2$ together with the nitrogen to which they are attached form a saturated heterocyclic ring having four to five carbon atoms, characterised in that rapamycin is acylated with an acylating agent containing an acylamino substituent having the configuration

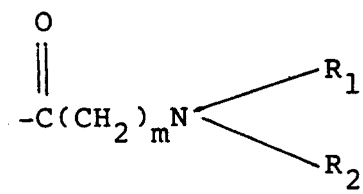

wherin $R_1$ and $R_2$ are as defined above,

and if required acidifying the product to form a pharmaceutically acceptable salt thereof.

**2.** A process as claimed in Claim 1 wherein $R_1$ and $R_2$ are each an alkyl radical of 1 to 3 carbon atoms.

**3.** A process as claimed in Claim 2 wherein $R_1$ and $R_2$ are methyl or ethyl.

**4.** A process as claimed in any one of Claims 1 to 3 wherein m is 1 or 2.

**5.** The process as claimed in Claim 1 in which the di-substituted derivative is prepared having substituent

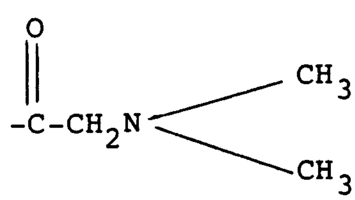

**6.** A process as claimed in Claim 5 in which the product is the methane sulphonate salt.

**7.** A process as claimed in Claim 1 wherein the acylating agent is the acid, the acid halide, the acid anhydride or an activated ester of said acylamino substituent.

**8.** A process as claimed in Claim 1 wherein the acylating agent is the acid of said acylamino substituent in the presence of a coupling agent.

**9.** A process as claimed in Claim 8 wherein the coupling agent is N, N'-dicyclohexylcarbodiimide, 1,1'-carbonyldiimidazole, diethylazodicarboxylate, 2,2'-dithiopyridine, or N,N-diisopropylcarbodiimide.

**10.** A process for preparing an injectable pharmaceutical composition which comprises bringing a water

soluble derivative of rapamycin as defined in any one of Claims 1 to 6 into a form suitable for therapeutic administration.

**Revendications**

**Revendications pour les Etats contractants suivants : BE, CH, DE, FR, IT, LI, LU, NL, SE**

1. Sel hydrosoluble, pharmaceutiquement acceptable, d'un dérivé de rapamycine qui est disubstitué en positions 28 et 43 de la rapamycine avec un substituant répondant à la formule structurale :

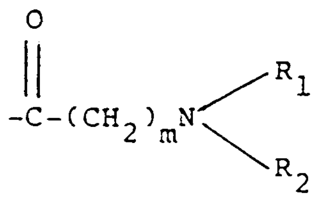

dans laquelle m représente un nombre entier de 1 à 3, $R_1$ et $R_2$ représentent chacun l'hydrogène ou un radical alkyle ayant 1 à 3 atomes de carbone, ou bien $R_1$ et $R_2$, conjointement avec l'atome d'azote auquel ils sont fixés, forment un noyau hétérocyclique saturé ayant quatre ou cinq atomes de carbone.

2. Dérivé de rapamycine suivant la revendication 1, dans lequel $R_1$ et $R_2$ représentent chacun un radical alkyle ayant 1 à 3 atomes de carbone.

3. Dérivé de rapamycine suivant la revendication 2, dans lequel $R_1$ et $R_2$ représentent chacun un groupe méthyle ou éthyle.

4. Dérivé de rapamycine suivant l'une quelconque des revendications 1 à 3, dans lequel m est égal à 1 ou 2.

5. Dérivé disubstitué suivant la revendication 1, dans lequel le substituant répond à la formule

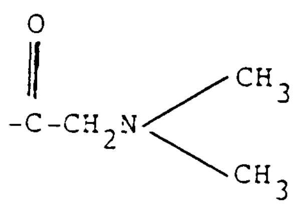

6. Méthanesulfonate du composé suivant la revendication 5.

7. Procédé de préparation d'un sel hydrosoluble, pharmaceutiquement acceptable, d'un dérivé de rapamycine qui est disubstitué en positions 28 et 43 par un substituant acylamino répondant à la formule structurale

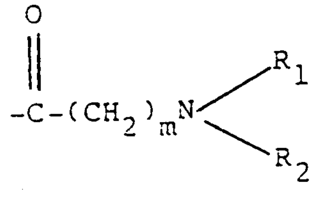

dans laquelle m, $R_1$ et $R_2$ répondent aux définitions suivant la revendication 1, qui consiste à acyler la rapamycine avec un agent d'acylation contenant le substituant acylamino répondant à la formule

structurale

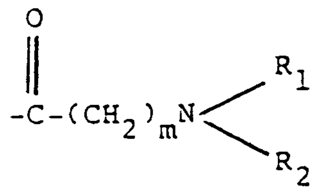

et, si nécessaire, à effectuer une acidification pour former un sel pharmaceutiquement acceptable.

**8.** Procédé suivant la revendication 7, dans lequel l'agent d'acylation est l'acide, l'halogénure d'acide, l'anhydride d'acide ou un ester activé dudit substituant acylamino.

**9.** Procédé suivant la revendication 8, dans lequel l'agent d'acylation est l'acide du substituant acylamino en présence d'un agent de couplage.

**10.** Procédé suivant la revendication 9, dans lequel l'agent de couplage est le N,N'-dicyclohexylcarbodiimide, le 1,1'-carbonyldiimidazole, l'azodicarboxylate de diéthyle, la 2,2'-dithiopyridine ou le N,N-diisopropylcarbodiimide.

**11.** Procédé suivant l'une quelconque des revendications 7 à 10, dans lequel $R_1$ et $R_2$ représentent chacun un groupe alkyle ayant 1 à 3 atomes de carbone.

**12.** Composition pharmaceutique injectable comprenant un dérivé hydrosoluble de rapamycine suivant l'une quelconque des revendications 1 à 6 et un véhicule pharmaceutiquement acceptable.

**Revendications pour les Etats contractants suivants : AT, GR, ES**

**1.** Procédé de préparation d'un sel hydrosoluble, pharmaceutiquement acceptable, d'un dérivé de rapamycine qui est disubstitué en positions 28 et 43 de la rapamycine avec un substituant acylamino répondant à la formule structurale :

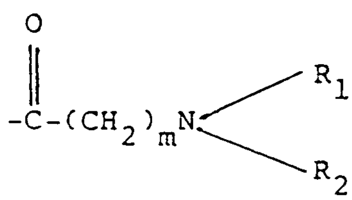

dans laquelle m représente un nombre entier de 1 à 3, $R_1$ et $R_2$ représentent chacun l'hydrogène ou un radical alkyle ayant 1 à 3 atomes de carbone, ou bien $R_1$ et $R_2$, conjointement avec l'atome d'azote auquel ils sont fixés, forment un noyau hétérocyclique saturé ayant 4 ou 5 atomes de carbone, caractérisé en ce que la rapamycine est acylée avec un agent d'acylation contenant un substituant acylamino répondant à la formule structurale

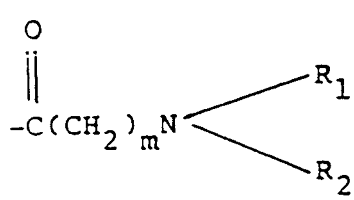

dans laquelle $R_1$ et $R_2$ répondent aux définitions précitées, et, le cas échéant, à acidifier le produit pour

former un sel pharmaceutiquement acceptable.

**2.** Procédé suivant la revendication 1, dans lequel $R_1$ et $R_2$ représentent chacun un radical alkyle ayant 1 à 3 atomes de carbone.

**3.** Procédé suivant la revendication 2, dans laquelle $R_1$ et $R_2$ représentent chacun un groupe méthyle ou éthyle.

**4.** Procédé suivant l'une quelconque des revendications 1 à 3, dans lequel m est égal à 1 ou 2.

**5.** Procédé suivant la revendication 1, dans lequel le dérivé disubstitué est préparé de manière à porter un substituant de formule

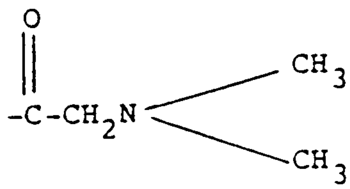

**6.** Procédé suivant la revendication 5, dans lequel le produit est le méthanesulfonate.

**7.** Procédé suivant la revendication 1, dans lequel l'agent d'acylation est l'acide, l'halogénure d'acide, l'anhydride d'acide ou un ester activé du substituant acylamino.

**8.** Procédé suivant la revendication 1, dans lequel l'agent d'acylation est l'acide du substituant acylamino en présence d'un agent de couplage.

**9.** Procédé suivant la revendication 8, dans lequel l'agent de couplage est le N,N'-dicyclohexylcarbodiimide, le 1,1'-carbonyldiimidazole, l'azodicarboxylate de diéthyle, la 2,2'-dithiopyridine ou le N,N-diisopropylcarbodiimide.

**10.** Procédé de préparation d'une composition pharmaceutique injectable, qui consiste à mettre un dérivé hydrosoluble de rapamycine suivant l'une quelconque des revendications 1 à 6 sous une forme convenable pour une administration thérapeutique.

**Patentansprüche**
**Patentansprüche für folgende Vertragsstaaten : BE, CH, DE, FR, IT, LI, LU, NL, SE**

**1.** Wasserlösliches pharmazeutisch annehmbares Salz eines Rapamycinderivates, das in den Stellungen 28 und 43 von Rapamycin mit einem Substituenten mit der Konfiguration

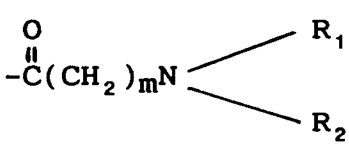 ,

worin m eine ganze Zahl von 1 bis 3 ist und $R_1$ und $R_2$ jeweils Wasserstoff oder einen Alkylrest mit 1 bis 3 Kohlenstoffatomen bedeuten oder $R_1$ und $R_2$ zusammen mit dem Stickstoffatom, an das sie gebunden sind, einen gesättigten heterocyclischen Ring mit 4 bis 5 Kohlenstoffatomen bilden, disubstituiert ist.

**2.** Rapamycinderivat nach Anspruch 1, worin $R_1$ und $R_2$ jeweils einen Alkylrest mit 1 bis 3 Kohlenstoffatomen bedeuten.

**3.** Rapamycinderivat nach Anspruch 2, worin $R_1$ und $R_2$ Methyl oder Äthyl bedeuten.

**4.** Rapamycinderivat nach einem der Ansprüche 1 bis 3, worin m 1 oder 2 ist.

**5.** Disubstituiertes Derivat nach Anspruch 1, worin der Substituent

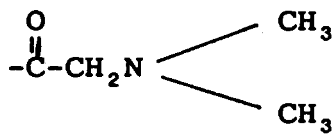

ist.

**6.** Methansulfonatsalz der Verbindung nach Anspruch 5.

**7.** Verfahren zum Herstellen eines wasserlöslichen pharmazeutisch annehmbaren Salzes eines Rapamycinderivates, das in den Stellungen 28 und 43 durch einen Acylaminosubstituenten mit der Konfiguration

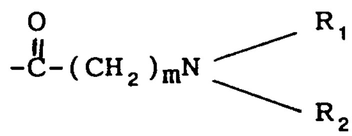

,

worin m, $R_1$ und $R_2$ wie in Anspruch 1 definiert sind, disubstituiert ist, das das Acylieren von Rapamycin mit einem Acylierungsmittel, das den Acylaminosubstituenten mit der Konfiguration

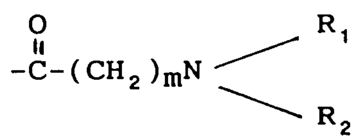

,

enthält, und, wenn erforderlich, das Ansäuern unter Bildung eines pharmazeutisch annehmbaren Salzes umfaßt.

**8.** Verfahren nach Anspruch 7, in dem das Acylierungsmittel die Säure, das Säurehalogenid, das Säureanhydrid oder ein aktivierter Ester des Acylaminosubstituenten ist.

**9.** Verfahren nach Anspruch 8, in dem das Acylierungsmittel die Säure des Acylaminosubstituenten in Anwesenheit eines Kopplungsmittels ist.

**10.** Verfahren nach Anspruch 9, in dem das Kopplungsmittel N,N'-Dicyclohexylcarbodiimid, 1,1'-Carbonyldiimidazol, Diäthylazodicarboxylat, 2,2'-Dithiopyridin oder N,N-Diisopropylcarbodiimid ist.

**11.** Verfahren nach einem der Ansprüche 7 bis 10, in dem $R_1$ und $R_2$ jeweils Alkyl mit 1 bis 3 Kohlenstoffatomen sind.

**12.** Injizierbare pharmazeutische Zusammensetzung umfassend ein wasserlösliches Rapamycinderivat nach einem der Ansprüche 1 bis 6 und einen pharmazeutisch annehmbaren Träger.

**Patentansprüche für folgende Vertragsstaaten : AT, GR, ES**

**1.** Verfahren zum Herstellen eines wasserlöslichen pharmazeutisch annehmbaren Salzes eines Rapamycinderivates, das in den Stellungen 28 und 43 von Rapamycin mit einem Acylaminosubstituenten mit der Konfiguration

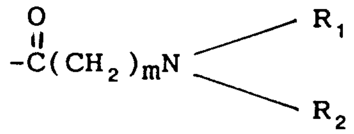

,

worin m eine ganze Zahl von 1 bis 3 ist und $R_1$ und $R_2$ jeweils Wasserstoff oder einen Alkylrest mit 1 bis 3 Kohlenstoffatomen bedeuten oder $R_1$ und $R_2$ zusammen mit dem Stickstoffatom, an das sie gebunden sind, einen gesättigten heterocyclischen Ring mit 4 bis 5 Kohlenstoffatomen bilden, disubstituiert ist, dadurch gekennzeichnet, daß Rapamycin mit einem Acylierungsmittel, das einen Acylaminosubstituenten mit der Konfiguration

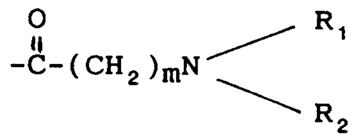

,

worin $R_1$ und $R_2$ wie oben definiert sind, enthält, acyliert wird und, wenn erforderlich, das Produkt unter Bildung eines pharmazeutisch annehmbaren Salzes hievon angesäuert wird.

**2.** Verfahren nach Anspruch 1, in dem $R_1$ und $R_2$ jeweils einen Alkylrest mit 1 bis 3 Kohlenstoffatomen bedeuten.

**3.** Verfahren nach Anspruch 2, in dem $R_1$ und $R_2$ Methyl oder Äthyl bedeuten.

**4.** Verfahren nach einem der Ansprüche 1 bis 3, in dem m 1 oder 2 ist.

**5.** Verfahren nach Anspruch 1, in dem das disubstituierte Derivat mit dem Substituenten

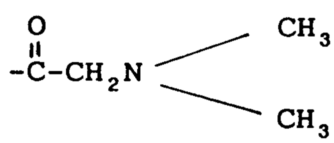

hergestellt wird.

**6.** Verfahren nach Anspruch 5, in dem das Produkt das Methansulfonatsalz ist.

**7.** Verfahren nach Anspruch 1, in dem das Acylierungsmittel die Saure, das Säurehalogenid, das Säureanhydrid oder ein aktivierter Ester des Acylaminosubstituenten ist.

**8.** Verfahren nach Anspruch 1, in dem das Acylierungsmittel die Säure des Acylaminosubstituenten in Anwesenheit eines Kopplungsmittels ist.

**9.** Verfahren nach Anspruch 8, in dem das Kopplungsmittel N,N'-Dicyclohexylcarbodiimid, 1,1'-Carbonyldiimidazol, Diäthylazodicarboxylat, 2,2'-Dithiopyridin oder N,N-Diisopropylcarbodiimid ist.

**10.** Verfahren zum Herstellen einer injizierbaren pharmazeutischen Zusammensetzung, das darin besteht, daß ein wasserlösliches Rapamycinderivat, wie in einem der Ansprüche 1 bis 6 definiert, in eine für therapeutische Verabreichung geeignete Form gebracht wird.